(19) **Europäisches Patentamt / European Patent Office / Office européen des brevets**

(11) **EP 1 791 482 B1**

(12) # EUROPEAN PATENT SPECIFICATION

(45) Date of publication and mention
of the grant of the patent:
**08.06.2011 Bulletin 2011/23**

(51) Int Cl.:
*A61B 18/00* (2006.01)  *A61B 18/14* (2006.01)
*A61B 17/22* (2006.01)  *A61N 7/02* (2006.01)
*B06B 1/06* (2006.01)

(21) Application number: **05826467.2**

(22) Date of filing: **13.09.2005**

(86) International application number:
**PCT/US2005/032935**

(87) International publication number:
**WO 2006/060053 (08.06.2006 Gazette 2006/23)**

(54) **ABLATION DEVICE WITH PHASED ARRAY ULTRASOUND TRANSDUCER**

ABLATIONSVORRICHTUNG MIT PHASENGESTEUERTEM ARRAY-ULTRASCHALLWANDLER

DISPOSITIF D'ABLATION A TRANSDUCTEUR ULTRASONIQUE A DEPHASAGE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **13.09.2004 US 609416 P**

(43) Date of publication of application:
**06.06.2007 Bulletin 2007/23**

(73) Proprietor: **Biosense Webster, Inc.
Diamond Bar, CA 91765 (US)**

(72) Inventor: **ROSINKO, Michael
Irwindale, California 91706 (US)**

(74) Representative: **Belcher, Simon James
Urquhart-Dykes & Lord LLP
Tower North Central
Merrion Way
Leeds LS2 8PA (GB)**

(56) References cited:
**WO-A-2005/009218    US-A- 4 135 109
US-A- 5 725 494    US-A- 6 117 101
US-A1- 2002 059 708**

## Description

### FIELD OF THE INVENTION

The present invention relates to a surgical device. More particularly, it relates to a device assembly and tissue ablation transducer having a plurality of transducer elements that can be operated out of phase to project and focus an acoustical energy beam on a particular treatment site.

### BACKGROUND OF THE INVENTION

Many local energy delivery devices and methods have been developed for treating the various abnormal tissue conditions in the body, and particularly for treating abnormal tissue along body space walls that define various body spaces in the body. For example, various devices have been disclosed with the primary purpose of treating or recanalizing atherosclerotic vessels with localized energy delivery. Several prior devices and methods combine energy delivery assemblies in combination with cardiovascular stent devices in order to locally deliver energy to tissue in order to maintain patency in diseased lumens such as blood vessels. Endometriosis, another abnormal wall tissue condition that is associated with the endometrial cavity and is characterized by dangerously proliferative uterine wall tissue along the surface of the endometrial cavity, has also been treated by local energy delivery devices and methods. Several other devices and methods have also been disclosed which use catheter-based heat sources for the intended purpose of inducing thrombosis and controlling hemorrhaging within certain body lumens such as vessels. Detailed examples of local energy delivery devices and related procedures such as those of the types described above are disclosed in the following references: U.S. Pat. No. 4,672,962 to Hershenson; U.S. Pat. No. 4,676,258 to InoKuchi et al.; U.S. Pat. No. 4,790,311 to Ruiz; U.S. Pat. No. 4,807,620 to Strul et al.; U.S. Pat. No. 4,998,933 to Eggers et al.; U.S. Pat. No. 5,035,694 to Kasprzyk et al.; U.S. Pat. No. 5,190,540 to Lee; U.S. Pat. No. 5,226,430 to Spears et al.; and U.S. Pat No. 5,292,321 to Lee; U.S. Pat. No. 5,449,380 to Chin; U.S. Pat. No. 5,505,730 to Edwards; U.S. Pat. No. 5,558,672 to Edwards et al.; and U.S. Pat. No. 5,562,720 to Stem et al.; U.S. Pat. No. 4,449,528 to Auth et al.; U.S. Pat. No. 4,522,205 to Taylor et al.; and U.S. Pat. No. 4,662,368 to Hussein et al.; U.S. Pat. No. 5,078,736 to Behl; and U.S. Pat. No. 5,178,618 to Kandarpa.

Other prior devices and methods electrically couple fluid to an ablation element during local energy delivery for treatment of abnormal tissues. Some such devices couple the fluid to the ablation element for the primary purpose of controlling the temperature of the element during the energy delivery. Other such devices couple the fluid more directly to the tissue-device interface either as another temperature control mechanism or in certain other known applications as a carrier or medium for the localized energy delivery. Detailed examples of ablation devices that use fluid to assist in electrically coupling electrodes to tissue are disclosed in the following references: U.S. Pat. No. 5,348,554 to Imran et al.; U.S. Pat. No. 5,423,811 to Imran et al.; U.S. Pat. No. 5,505,730 to Edwards; U.S. Pat. No. 5,545,161 to Imran et al.; U.S. Pat No. 5,558,672 to Edwards et al.; U.S. Pat. No. 5,569,241 to Edwards; U.S. Pat No. 5,575,788 to Baker et al.; U.S. Pat. No. 5,658,278 to Imran et al.; U.S. Pat No. 5,688,267 to Panescu et al.; U.S. Pat. No. 5,697,927 to Imran et al.; U.S. Pat. No. 5,722,403 to McGee et al.; U.S. Pat No. 5,769,846; and PCT Patent Application Publication No. WO 97/32525 to Pomeranz et al.; and PCT Patent Application Publication No. WO 98/02201 to Pomeranz et al. Atrial Fibrillation.

Cardiac arrhythmias, and atrial fibrillation in particular, persist as common and dangerous medical aliments associated with abnormal cardiac chamber wall tissue, and are often observed in elderly patients. In patients with cardiac arrhythmia, abnormal regions of cardiac tissue do not follow the synchronous beating cycle associated with normally conductive tissue in patients with sinus rhythm. Instead, the abnormal regions of cardiac tissue aberrantly conduct to adjacent tissue, thereby disrupting the cardiac cycle into an asynchronous cardiac rhythm. Such abnormal conduction is known to occur at various regions of the heart, such as, for example, in the region of the sino-atrial (SA) node, along the conduction pathways of the atrioventricular (AV) node and the Bundle of His, or in the cardiac muscle tissue forming the walls of the ventricular and atrial cardiac chambers.

Cardiac arrhythmias, including atrial arrhythmia, may be of a multiwavelet reentrant type, characterized by multiple asynchronous loops of electrical impulses that are scattered about the atrial chamber and are often self-propagating. In the alternative or in addition to the multiwavelet reentrant type, cardiac arrhythmias may also have a focal origin, such as when an isolated region of tissue in an atrium fires autonomously in a rapid, repetitive fashion. Cardiac arrhythmia, including atrial fibrillation, may be generally detected using the global technique of an electrocardiogram (EKG). More sensitive procedures of mapping the specific conduction along the cardiac chambers have also been disclosed, such as, for example, in U.S. Pat. No. 4,641,649 to Walinsky et al. and in PCT Patent Application Publication No. WO 96/32897 to Desai.

A host of clinical conditions can result from the irregular cardiac function and resulting hemodynamic abnormalities associated with atrial fibrillation, including stroke, heart failure, and other thromboembolic events. In fact, atrial fibrillation is believed to be a significant cause of cerebral stroke, wherein the abnormal hemodynamics in the left atrium caused by the fibrillatory wall motion precipitate the formation of thrombus within the atrial chamber. A thromboembolism is ultimately dislodged into the left ventricle that thereafter pumps the embolism into the cerebral circulation where a stroke results. Accordingly, numerous procedures for treating atrial arrhythmias have been developed, including pharmacological, surgical, and catheter ablation procedures.

Several pharmacological approaches intended to remedy or otherwise treat atrial arrhythmias have been disclosed, such as, for example, those approaches disclosed in the following references: U.S. Pat. No. 4,673,563 to Berne et al.; U.S. Pat. No. 4,569,801 to Molloy et al.; and "Current Management of Arrhythmias" (1991) by Hindricks, et al. Such pharmacological solutions, however, are not generally believed to be entirely effective in many cases, and are even believed in some cases to result in proarrhythmia and long term inefficacy.

Several surgical approaches have also been developed with the intention of treating atrial fibrillation. One particular example is known as the "maze procedure," as is disclosed by Cox, J. L et al. in The surgical treatment of atrial fibrillation. I. Summary" Thoracic and Cardiovascular Surgery 101(3), pp. 402-405 (1991); and also by Cox, J L in "The surgical treatment of atrial fibrillation. IV. Surgical Technique", Thoracic and Cardiovascular Surgery 101(4), pp. 584-592 (1991). In general, the "maze" procedure is designed to relieve atrial arrhythmia by restoring effective atrial systole and sinus node control through a prescribed pattern of incisions about the tissue wall. In the early clinical experiences reported, the "maze" procedure included surgical incisions in both the right and the left atrial chambers. However, more recent reports predict that the surgical "maze" procedure may be substantially efficacious when performed only in the left atrium. See Sueda et al., "Simple Left Atrial Procedure for Chronic Atrial Fibrillation Associated With Mitral Valve Disease" (1996).

The "maze procedure" as performed in the left atrium generally includes forming vertical incisions from the two superior pulmonary veins and terminating in the region of the mitral valve annulus, traversing the region of the inferior pulmonary veins en route. An additional horizontal line also connects the superior ends of the two vertical incisions. Thus, the atrial wall region bordered by the pulmonary vein ostia is isolated from the other atrial tissue. In this process, the mechanical sectioning of atrial tissue eliminates the arrhythmogenic conduction from the boxed region of the pulmonary veins to the rest of the atrium by creating conduction blocks within the aberrant electrical conduction pathways. Other variations or modifications of this specific pattern just described have also been disclosed, all sharing the primary purpose of isolating known or suspected regions of arrhythmogenic origin or propagation along the atrial wall.

While the "maze" procedure and its variations as reported by Dr. Cox and others have met some success in treating patients with atrial arrhythmia, its highly invasive methodology is believed to be prohibitive in most cases. However, these procedures have provided a guiding principle that electrically isolating faulty cardiac tissue may successfully prevent atrial arrhythmia, and particularly atrial fibrillation caused by arrhythmogenic conduction arising from the region of the pulmonary veins.

Less invasive catheter-based approaches to treat atrial fibrillation have been disclosed which implement cardiac tissue ablation for terminating arrhythmogenic conduction in the atria. Examples of such catheter-based devices and treatment methods have generally targeted atrial segmentation with ablation catheter devices and methods adapted to form linear or curvilinear lesions in the wall tissue that defines the atrial chambers. Some specifically disclosed approaches provide specific ablation elements that are linear over a defined length intended to engage the tissue for creating the linear lesion. Other disclosed approaches provide shaped or steerable guiding sheaths, or sheaths within sheaths, for the intended purpose of directing tip ablation catheters toward the posterior left atrial wall such that sequential ablations along the predetermined path of tissue may create the desired lesion. In addition, various energy delivery modalities have been disclosed for forming atrial wall lesions, and include use of microwave, laser, ultrasound, thermal conduction, and more commonly, radiofrequency energies to create conduction blocks along the cardiac tissue wall.

Detailed examples of ablation device assemblies and methods for creating lesions along an atrial wall are disclosed in the following U.S. Patent references: U.S. Pat. No. 4,898,591 to Jang et al; U.S. Pat. No. 5,104,393 to Isner et al.; U.S. Pat. Nos. 5,427,119; 5,487,385 to Avitall; U.S. Pat. No. 5,497,119 to Swartz et al.; U.S. Pat. No. 5,545,193 to Fleischman et al.; U.S. Pat. No. 5,549,661 to Kordis et al.; U.S. Pat. No. 5,575,810 to Swanson et al.; U.S. Pat. No. 5,564,440 to Swartz et al.; U.S. Pat. No. 5,592,609 to Swanson et al. ; U.S. Pat. No. 5,575,766 to Swartz et al.; U.S. Pat. No. 5,582,609 to Swanson; U.S. Pat. No. 5,617,854 to Munsif; U.S. Pat. No 5,687,723 to Avitall; U.S. Pat. No. 5,702,438 to Avitall. Other examples of such ablation devices and methods are disclosed in the following PCT Patent Application Publication Nos.: WO 93/20767 to Stem et al.; WO 94/21165 to Kordis et al.; WO 96/10961 to Fleischman et al.; WO 96/26675 to Klein et al.; and WO 97/37607 to Schaer. Additional examples of such ablation devices and methods are disclosed in the following published articles: "Physics and Engineering of Transcatheter Tissue Ablation". Avitall et al., Journal of American College of Cardiology, Volume 22, No. 3: 921-932 (1993); and "Right and Left Atrial Radiofrequency Catheter Therapy of Paroxysmal Atrial Fibrillation," Haissaguerre, et al., Journal of Cardiovascular Electrophysiology 7(12), pp. 1132-1144 (1996).

In addition to those known assemblies summarized above, additional tissue ablation device assemblies have been recently developed for the specific purpose of ensuring firm contact and consistent positioning of a linear ablation element along a length of tissue by anchoring the element at least at one predetermined location along that length, such as in order to form a "maze"-type lesion pattern in the left atrium. One example of such assemblies is that disclosed in U.S. Pat. No. 5,971,983, issued Oct. 26, 1999. The assembly includes an anchor at each of two ends of a linear ablation element in order to secure those ends to each of two predetermined locations along a left atrial wall, such as at two adjacent pulmonary veins, so that tissue may be ablated along the length of tissue extending there between.

In addition to attempting atrial wall segmentation with long linear lesions for treating atrial arrhythmia, other ablation device and method have also been disclosed which are intended to use expandable members such as balloons to ablate cardiac tissue. Some such devices have been disclosed primarily for use in ablating tissue wall regions along the cardiac chambers. Other devices and methods have been disclosed for treating abnormal conduction of the left-sided accessory pathways, and in particular associated with "Wolff-Parkinson-White" syndrome--various such disclosures use a balloon for ablating from within a region of an associated coronary sinus adjacent to the desired cardiac tissue to ablate. Further more detailed examples of devices and methods such as of the types just described are variously disclosed in the following published references: Fram et al., in "Feasibility of RF Powered Thermal Balloon Ablation of Atrioventricular Bypass Tracts via the Coronary Sinus: In vivo Canine Studies," PACE, Vol. 18, p 1518-1530 (1995); "Long-term effects of percutaneous laser balloon ablation from the canine coronary sinus", Schuger CD et al., Circulation (1992) 86:947-954; and "Percutaneous laser balloon coagulation of accessory pathways", McMath L P et al., Diagn Ther Cardiovasc Interven 1991; 1425:165-171.

Arrhythmias Originating from Foci in Pulmonary Veins

Various modes of atrial fibrillation have also been observed to be focal in nature, caused by the rapid and repetitive firing of an isolated center within cardiac muscle tissue associated with the atrium. Such foci may act as either a trigger of atrial fibrillatory paroxysmal or may even sustain the fibrillation. Various disclosures have suggested that focal atrial arrhythmia often originates from at least one tissue region along one or more of the pulmonary veins of the left atrium, and even more particularly in the superior pulmonary veins.

Less-invasive percutaneous catheter ablation techniques have been disclosed which use end-electrode catheter designs with the intention of ablating and thereby treating focal arrhythmias in the pulmonary veins. These ablation procedures are typically characterized by the incremental application of electrical energy to the tissue to form focal lesions designed to terminate the inappropriate arrhythmogenic conduction.

One example of a focal ablation method intended to treat focal arrhythmia originating from a pulmonary vein is disclosed by Haissaguerre, et al. in "Right and Left Atrial Radiofrequency Catheter Therapy of Paroxysmal Atrial Fibrillation" in Journal of Cardiovascular Electrophysiology 7(12), pp. 1132-1144 (1996). Haissaguerre, et al. discloses radiofrequency catheter ablation of drug-refractory paroxysmal atrial fibrillation using linear atrial lesions complemented by focal ablation targeted at arrhythmogenic foci in a screened patient population. The site of the arrhythmogenic foci were generally located just inside the superior pulmonary vein, and the focal ablations were generally performed using a standard 4 mm tip single ablation electrode.

Another focal ablation method of treating atrial arrhythmias is disclosed in Jais et al., "A focal source of atrial fibrillation treated by discrete radiofrequency ablation," Circulation 95:572- 576 (1997). Jais et al. discloses treating patients with paroxysmal arrhythmias originating from a focal source by ablating that source. At the site of arrhythmogenic tissue, in both right and left atria, several pulses of a discrete source of radiofrequency energy were applied in order to eliminate the fibrillatory process.

Other assemblies and methods have been disclosed addressing focal sources of arrhythmia in pulmonary veins by ablating circumferential regions of tissue either along the pulmonary vein, at the ostium of the vein along the atrial wall, or encircling the ostium and along the atrial wall. More detailed examples of device assemblies and methods for treating focal arrhythmia as just described are disclosed in PCT Patent Application Publication No. WO 99/02096 to Diederich et al., and also in the following pending U.S. patent and patent applications: U.S. Pat. No. 6,024,740, issued on Feb. 15, 2000 to Michael D. Lesh et al., for "Circumferential Ablation Device Assembly"; U.S. Pat. No. 6,012,457, issued on Jan. 11, 2000 to Michael D. Lesh, for "Device and Method for Forming a Circumferential Conduction Block in a Pulmonary Vein"; U.S. Pat. No. 6,117,101 issued on Sept. 12, 2000 to Chris J. Diederich et al., for "Circumferential Ablation Device Assembly"; and U.S. Ser. No. 09/260,316 for "Device and Method for Forming a Circumferential Conduction Block in a Pulmonary Vein" to Michael D. Lesh.

Another specific device assembly and method which is intended to treat focal atrial fibrillation by ablating a circumferential region of tissue between two seals in order to form a conduction block to isolate an arrhythmogenic focus within a pulmonary vein is disclosed in U.S. Pat. No. 5,938,660 and a related PCT Patent Application Publication No. WO 99/00064.

US-4135109 discusses a high powered cylindrical piezoelectric transducer. The active element is a radially poled piezoceramic having a surface into which a square thread is spirally cut so that only a pair of electrical leads are needed to excite the whole assembly.

WO-2005/009218 forms prior art under Article 54(3) EPC. It relates to an ablation device with a spiral array transducer. An outer electrode is segmented by grooves into a small number of intertwined helical elements.

## SUMMARY OF THE INVENTION

The present invention relates to a device assembly and tissue ablation transducer having a plurality of discrete serially arranged transducer elements that can be operated out of phase from one another to orient the acoustical energy beam forward or backward in the longitudinal direction. The transducer elements may also be driven by a signal that has a common frequency, but where each element has a phase that is chosen to provide an acoustic energy beam that has an additive effect at a predetermined distance from the transducers.

According to the invention there is provided a cylindrical ultrasound transducer as defined in appended claim 1. It comprises a cylindrical inner electrode, a cylindrical piezoelectric material disposed over the inner electrode, and a cylindrical outer electrode disposed over the cylindrical piezoelectric material. The cylindrical outer electrode has circumferential grooves separating the outer electrode into a plurality of discrete serially arranged transducer elements.

In another embodiment of the present invention, a cylindrical ultrasound transducer comprises a cylindrical inner electrode, a cylindrical piezoelectric material disposed over the inner electrode, and a cylindrical outer electrode disposed over the cylindrical piezoelectric material. Circumferential grooves cut through the outer electrode and at least a portion of the cylindrical piezoelectric material separate the transducer into a plurality of functionally discrete serially arranged transducer segments.

## BRIEF DESCRIPTION OF THE DRAWINGS

Figure 1A is a perspective representation showing an example of a circular ablation path.

Figure 1B is a perspective representation showing an example of an elliptical ablation path.

Figure 1C is a perspective representation showing an example of an irregular ablation path.

Figure 1D is a perspective representation showing an example of a stepped ablation path.

Figure 2A is a perspective view showing an ablation catheter operably connected to an ablation control system and a position sensing system according to one embodiment of the present invention. An expandable member of the catheter is illustrated in an expanded state.

Figure 2B is a perspective view showing the details of an ablation member in the expanded state at a distal end of the ablation catheter of Figure 2A according to one embodiment of the present invention.

Figure 3A is a transverse cross-section view showing the construction of a cylindrical ultrasonic transducer having inner and outer electrodes.

Figure 3B is a perspective view of an ultrasound transducer in isolation, showing the electrical leads coupled to the transducer.

Figure 3C is a side view of an ablation catheter showing the collimated radial acoustical energy beam paths when the ablation device is place in a body lumen, such as a pulmonary vein.

Figure 3D is a side view of a catheter showing the collimated radial acoustical energy beam paths when the ablation device is placed at the juncture between a body lumen and a body cavity, such as a pulmonary vein ostium,

Figure 4A is side view of an ablation catheter that doesn't form part of the invention having a plurality of cylindrical ultrasound transducers.

Figure 4B is a perspective view of a single cylindrical ultrasound transducer from Figure 4A.

Figure 5A is a perspective view of an ablation catheter having an ultrasonic transducer segmented into discrete transducer elements according to one embodiment of the present invention.

Figure 5B is a section view taken through section 5B-5B in Figure 5A showing an ultrasonic transducer segmented into discrete transducer elements according to one embodiment of the present invention.

Figure 6A is a perspective view of an ablation catheter having an ultrasonic transducer segmented into discrete transducer elements according to one embodiment of the present invention.

Figure 6B is a section view taken through section 5B-5B in Figure 5A showing an ultrasonic transducer segmented into discrete transducer elements according to one embodiment of the present invention.

Figure 7A is a schematic representation illustrating a fixed phased delay (phase shift) for a plurality of sinusoidal input signals driving an array of transducers according to one embodiment of the present invention.

Figure 7B is a schematic representation showing the resultant cumulative acoustic energy beam emanating from the transducers at an angle relative to the longitudinal axis according to one embodiment of the present invention.

Figure 8A is a graphical representation of an amplitude profile for an acoustical energy beam emanating from an array of transducers according to one embodiment of the present invention.

Figure 8B is a schematic representation showing the cumulative acoustic energy beam having an additive or amplified effect at a predetermined distance from the transducer according to one embodiment of the present Invention.

Figure 9A is a schematic representation showing the cumulative acoustic energy beam being projected forward according to one embodiment of the present invention.

# DETAILED DESCRIPTION OF THE INVENTION

## Definitions of Terms

The following terms will have the following meanings throughout this specification.

The terms "body space," including derivatives thereof, is herein intended to mean any cavity or lumen within the body that is defined at least in part by a tissue wall. For example, the cardiac chambers, the uterus, the regions of the gastrointestinal tract, and the arterial or venous vessels are all considered illustrative examples of body spaces within the intended meaning.

The terms "circumference" or "circumferential", including derivatives thereof, as used herein include a substantially continuous path or line that forms an outer border or perimeter that surrounds and thereby defines an enclosed region of space. Such a continuous path starts at one location along the outer border or perimeter, and translates along the outer border or perimeter until it is completed at the original starting location to enclose the defined region of space. The related term "circumscribe," including derivatives thereof, as used herein includes a surface to enclose, surround, or encompass a defined region of space. Therefore, a continuous line which is traced around a region of space and which starts and ends at substantially the same location "circumscribes" the region of space and has a "circumference" which includes the distance the line travels as it translates along the path circumscribing the space.

Still further, a circumferential path or element may include one or more of several shapes, and may be for example circular, oblong, ovular, elliptical, or otherwise planar enclosures. A circumferential path may also be three dimensional, such as for example two opposite-facing semi-circular paths in two different parallel or off-axis planes that are connected at their ends by line segments bridging between the planes.

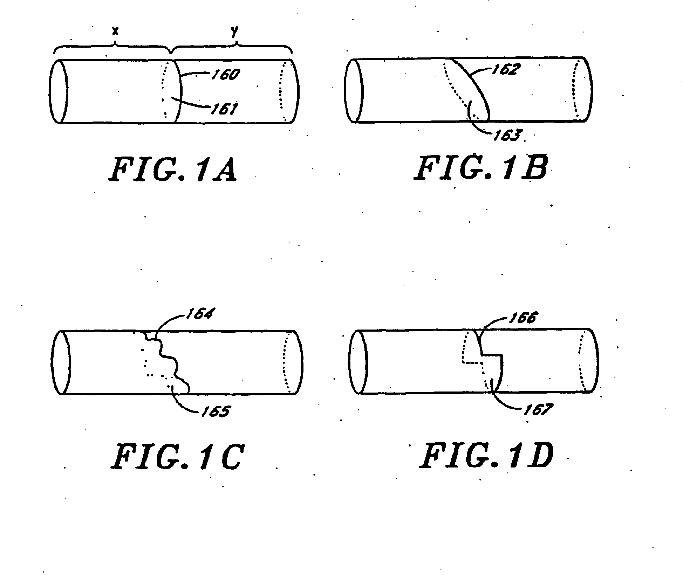

For purpose of further illustration and example, Figures 1A-1D show circumferential paths 160, 162, 164, and 166, respectively. Each path 160, 162, 164, 166 translates along a portion of a body space, for example a pulmonary vein wall, and circumscribes a defined region of space, shown at 161, 163, 165, and 167, respectively, each circumscribed region of space being a portion of the body space. However, the circumferential path does not necessarily have to translate along a tubular structure as shown, and other geometric structures are also contemplated, such as along the atrial wall in the atrium of a heart.

The term "transect", including derivatives thereof, as used herein includes a way to divide or separate a region of space into isolated regions. Thus, each of the regions circumscribed by the circumferential paths shown in Figures 1A-D transects the respective body space, for example the pulmonary vein ostium, including its lumen and its wall, to the extent that the respective body space is divided into a first longitudinal region located on one side of the transecting region, shown for example at region "X" in Figure 1A, and a second longitudinal region on the other side of the transecting plane, shown for example at region "Y" also in Figure 1A. Similarly, a circumferential path along other structures, such as the atrial wall around the pulmonary vein ostium will transect the pulmonary vein from the atrium.

Therefore, a "circumferential conduction block" according to the present Invention is formed along a region of tissue that follows a circumferential path, circumscribing the tissue region and transecting the region of tissue relative to electrical conduction along the circumferential path. By way of example, the transecting circumferential conduction block therefore isolates electrical conduction between the left atrium and a pulmonary vein.

The terms "ablate" or "ablation," including derivatives thereof, are hereafter intended to include the substantial altering of the mechanical, electrical, chemical, or other structural nature of tissue. In the context of ablation applications shown and described with reference to the variations of the illustrative device below, "ablation" is intended to include sufficient altering of tissue properties to substantially block conduction of electrical signals from or through the ablated cardiac tissue.

The term "element" within the context of "ablation element" is herein intended to include a discrete element, such as an ultrasonic transducer, or a plurality of discrete elements, such as a plurality of spaced ultrasonic transducers, which are positioned so as to collectively ablate a region of tissue.

Therefore, an "ablation element" according to the defined terms can include a variety of specific structures adapted to ablate a defined region of tissue. For example, one suitable ablation element for use in the present invention may be formed, according to the teachings of the embodiments below, from an "energy emitting" type of structure which is adapted to emit energy sufficient to ablate tissue when coupled to and energized by an energy source. One particular suitable "energy emitting" ablation element for use in the present invention may therefore include, for example an ultrasonic element such as an ultrasound crystal element which is adapted to emit ultrasonic sound waves sufficient to ablate tissue when coupled to a suitable excitation source.

## Embodiments of the Invention

The following describes ablation devices of a medical device system. The disclosed devices may include a position monitoring system that allows a clinician to precisely locate a distal end of the medical device within a body space by using feedback information provided by the system. Such feedback information is indicative of the position of the distal end of the medical device within the body space. The following devices of the position monitoring system are particularly well suited for applications involving positioning an ablation member at an area where a pulmonary vein extends from a left atrium, including the atrial back wall, and relative to a targeted circumferential region of tissue within the area, and therefore these devices are described in this context. Various aspects of the present invention, however, can be readily adapted by those skilled in the art for applications involving positioning medical articles within other body spaces.

In the context of the illustrative application, catheter-based cardiac arrhythmia therapies generally involve introducing an ablation catheter into a cardiac chamber, such as in a percutaneous transluminal procedure, wherein an ablation element on the catheter's distal end portion is positioned at or adjacent to the aberrant conductive tissue. The ablation element is used to ablate the targeted tissue thereby creating a lesion.

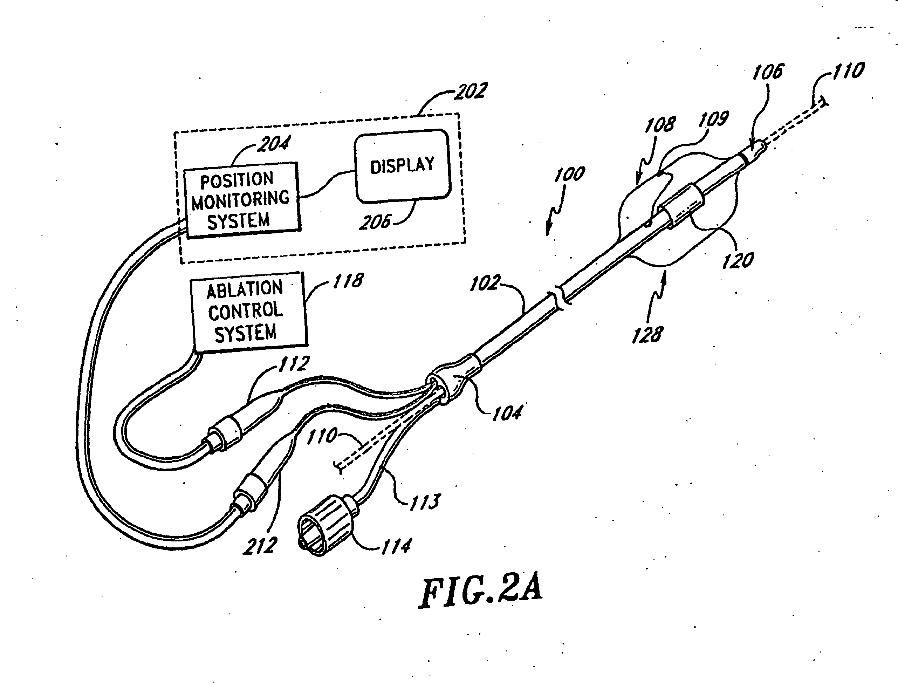

Figure 2A shows an exemplary ablation catheter assembly 100 operably connected through an electrical connector 112 to an ablation control system 118. The catheter assembly 100 includes an elongated delivery member 102 with a proximal end portion 104 and a distal end portion 106. The distal end portion 106 supports an ablation member 128 including an ablation element 120 and an anchor mechanism 108. In one preferred embodiment (illustrated in Figure 2A), the anchor mechanism 108 is an expandable member. The expandable member can also include a sensor 109 that is explained below. Ablation element 120 may include a single ultrasonic transducer, or a plurality of ultrasonic transducers.

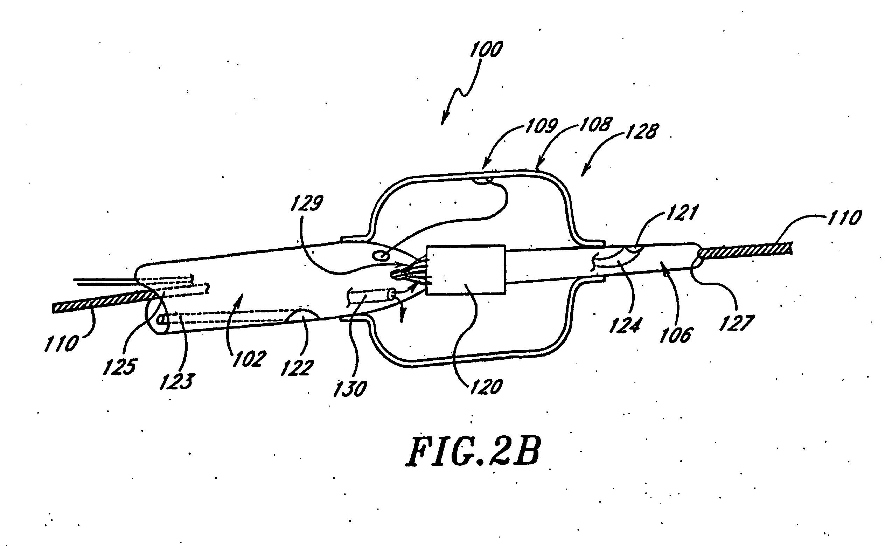

The delivery member 102 desirably includes a plurality of lumens (some of which are illustrated in Figure 2B). Various wires and electrical leads are routed to the distal end portion 106 through at least some of these lumens. In a preferred device, these lumens generally run the length of the delivery member 102; however, for some applications, the lumens can be shorter. In one example, a guidewire 110 runs through a lumen in the delivery member 102 from the proximal end portion 104 to the distal end portion 106. The proximal end portion 104 also connects through a tube 113 to a screw connector 114. By introducing fluid into the tube 113 through the screw connector 114, a physician can inflate the expandable member 108, as known in the art.

In some modes of the catheter assembly, as seen in Figure 2B, the delivery member 102 includes a distal port 121, which is distal to an ablation member 128. In addition, there is a proximal port 122, which is provided proximal of the ablation member 128. The proximal port 122 connects to a proximal port lumen 123, and the distal port 121 connects to a distal port lumen 124. The distal port 121 allows the clinician to introduce fluids into the patient, take fluid samples from the patient, and take fluid pressure reading on the distal side of the ablation member 128. Similarly, the proximal port 122 allows the clinician to introduce fluids into the patient, take fluid samples from the patient, and take fluid pressure reading on the proximal side of the ablation member 128. These ports 121, 122 and lumens 123 and 124 are particularly useful when pressure or X-ray positioning techniques are employed, as explained below; however, the catheter assembly 100 need not include such ports and lumens when only an A-mode or Doppler position monitoring system is used with the catheter assembly.

In the illustrated device, the delivery member 102 also includes a guidewire lumen 125 that is sized to track over the guidewire 110. The lumen 125 terminates at a distal port 127 located on the distal end 106 of the delivery member 102.

When constructed for use in transeptal left atrial ablation procedures, the delivery member 102 desirably has an outer diameter provide within the range of from about 5 French to about 10 French, and more preferably from about 7 French to about 9 French. The guidewire lumen 125 preferably is adapted to slideably receive guidewires ranging from about $2.54 \cdot 10^{-2}$ cm to about $9.652 \cdot 10^{-2}$ cm (from about 0.010 inch to about 0.038 inch) in diameter, and preferably is adapted for use with guidewires ranging from about $4.572 \cdot 10^{-2}$ cm to about $8.89 \cdot 10^{-2}$ cm (from about 0.018 inch to about 0.035 inch) in diameter. Where a $8.89 \cdot 10^{-2}$ cm (0.035 inch) guidewire is to be used, the guidewire lumen 125 preferably has an inner diameter of $10.16 \cdot 10^{-2}$ cm to about $10.67 \cdot 10^{-2}$ cm (0.040 inch to about 0.042 inch). In addition, where the delivery member 102 includes an inflation lumen 130 for use with an inflatable balloon (a preferred form of the expandable member 108), the inflation lumen 130 preferably has an inner diameter of about $5.08 \cdot 10^{-2}$ cm (0.020 inch) in order to allow for rapid deflation times, although this may vary based upon the viscosity of inflation medium used, length of the lumen 130, and other dynamic factors relating to fluid flow and pressure.

In addition to providing the requisite lumens and support for the ablation member 128, the delivery member 102 for the illustrative application also is adapted to be introduced into the left atrium such that the distal end portion 106 can be placed within the pulmonary vein ostium in a percutaneous translumenal procedure, and even more preferably in a transeptal procedure as otherwise herein provided. Therefore, the distal end portion 106 is preferably flexible and adapted to track over and along a guidewire seated within the targeted pulmonary vein.

In a further construction, the proximal end portion 104 is adapted to be at least 30% more stiff than the distal end portion 106. According to this relationship, the proximal end portion 104 may be suitably adapted to provide push transmission to the distal end portion 106 while the distal end portion 106 is suitably adapted to track through bending anatomy during in vivo delivery of the distal end portion 106 of the device into the desired ablation region.

Notwithstanding the specific device construction just described, other delivery mechanisms for delivering the ablation member 128 to the desired ablation region are also contemplated. For example, while the Figure 2A variation is shown as an "over-the-wire" catheter construction, other guidewire tracking designs are suitable substitutes, such as, for example, catheter devices that are known as "rapid exchange" or "monorail" variations, wherein the guidewire is only housed coaxially within a lumen of the catheter in the distal region of the catheter. In another example, a deflectable tip design may also be a suitable substitute to independently select a desired pulmonary vein and direct the transducer assembly into the desired location for ablation. Further to this latter variation, the guidewire lumen and guidewire of the variation depicted in Figure 2A. may be replaced with a "pullwire" lumen and associated fixed pullwire which is adapted to deflect the catheter tip by applying tension along varied stiffness transitions along the catheter's length. Still further to this pullwire variation, acceptable pullwires may have a diameter within the range from about $2.03 \cdot 10^{-2}$ cm to about $5.08 \cdot 10^{-2}$ cm (from about 0.008 inch to about 0.020 inch), and may further include a taper, such as, for example, a tapered outer diameter from about $5.08 \cdot 10^{-2}$ cm to about $2.03 \cdot 10^{-2}$ cm (from about 0.020 inch to about 0.008 inch).

As discussed above, the distal end portion 106 of the delivery member supports an ablation member 128. The ablation member 128 includes an expandable member 108 and an ablation element 120. The expandable member 108 cooperates with the ablation element 120 to position and anchor the ablation element 120 relative to a circumferential region of tissue. Regions of tissue targeted for ablation may include, for example, a location where a pulmonary vein extends from the left atrium, including the back atrial wall of the left atrium, the pulmonary vein ostium or the pulmonary vein.

In the illustrated device, the expandable member 108 is an inflatable balloon. The balloon has a diameter in a collapsed state roughly the same as the outer diameter of the delivery member distal end portion 106. The balloon 108 can be expanded to a diameter generally matching the diameter of the circumferential region of tissue, and may be expandable to a plurality of expanded positions in order to work with the atrium, pulmonary vein ostia and/or pulmonary veins of various sizes. It is understood, however, that the ablation catheter assembly can also include other types of expandable members, such as, for example baskets, cages and like expandable structures.

The expandable balloon 108 may be constructed from a variety of known materials, although the balloon preferably is adapted to conform to the contour of a pulmonary vein ostium and/or pulmonary vein lumenal wall. For this purpose, the balloon material can be of the highly compliant variety, such that the material elongates upon application of pressure and takes on the shape of the body lumen or space when fully inflated. Suitable balloon materials include elastomers, such as, for example, but without limitation, silicone, latex, or low durometer polyurethane (for example a durometer of about 80 A).

In addition, or in the alternative to constructing the balloon of highly compliant material, the balloon can be formed to have a predefined fully inflated shape (i.e., be preshaped) to generally match the anatomic shape of the body lumen in which the balloon is inflated. For instance, the balloon can have a distally tapering shape to generally match the shape of a pulmonary vein ostium, and/or can include a bulbous proximal end to generally match a transition region of the atrium posterior wall adjacent to the pulmonary vein ostium. In this manner, the desired seating within the irregular geometry of a pulmonary vein or vein ostium can be achieved with both compliant and non-compliant balloon variations.

Notwithstanding the alternatives which may be acceptable as just described, the balloon is preferably constructed to exhibit at least 300% expansion at 3 atmospheres of pressure, and more preferably to exhibit at least 400% expansion at that pressure. The term "expansion" is herein intended to mean the balloon outer diameter after pressurization divided by the balloon inner diameter before pressurization, wherein the balloon inner diameter before pressurization is taken after the balloon is substantially filled with fluid in a taut configuration. In other words, "expansion" is herein intended to relate to the change in diameter that is attributable to the material compliance in a stress/strain relationship. In one more detailed construction, which is believed to be suitable for use in most conduction block procedures in the region of the pulmonary veins, the balloon is adapted to expand under a normal range of pressure such that its outer diameter may be adjusted from a radially collapsed position of about 5 millimeters to a radially expanded position of about 2.5 centimeters (or approximately 500% expansion).

The ablation element 120 cooperates with the expandable member 108 such that the ablation element 120 is held in a generally fixed position relative to the target circumferential region of tissue. The ablation element can be located outside or inside the expandable member, or can be located at least partially outside the expandable member. The ablation element, in some forms, also includes a portion of the expandable member. For instance, the ablation catheter assembly in Figures 2A and 2B may include a plurality of ultrasonic transducers located within the expandable member 108. In one device, the ultrasonic transducers excite a portion of the expandable member 108 during ablation. The specific construction of the ultrasonic transducer and the associated construction of the delivery member shaft that supports the transducer, is described below.

Figure 2B shows details of the distal end portion 106 of the catheter assembly 100 and, in particular, shows the ablation element 120 located longitudinally along an axial centerline of the delivery member 102. A plurality of wires 129 connect the ablation element 120 to a connector 112 at the proximal end of the catheter (shown in Figure 2A). The connector 112 is coupled to a corresponding cable of the ablation control system 118. If the ablation element 120 includes more than one transducer, the conductor lead can connect to all of the transducers, or separate conductors can be used so as to allow for independent control of each transducer under some modes of operation.

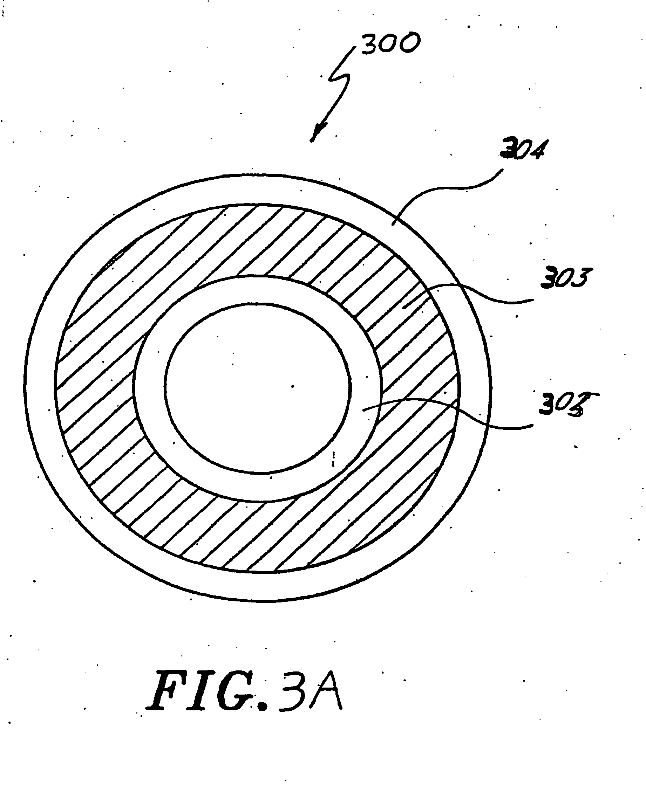

In a preferred embodiment, the ablation element 120 is comprised of a plurality of cylindrical ultrasonic transducers arranged serially along the delivery member 102. A cross-section view showing the construction of a typical single cylindrical ultrasonic transducer 300 having a cylindrical inner electrode 305, a cylindrical outer electrode 304, and a cylindrical piezoelectric material 303 between the electrodes is shown in Figure 3A. The piezoelectric material 303 is a suitable material, such as, for example quartz, PZT, and the like, that exhibits a change in physical dimension in response to an impressed voltage. The piezoelectric material 303 is oriented such that when a voltage is impressed between the electrodes 305 and 304, the thickness of the piezoelectric material 303 changes slightly. When the polarity of the impressed voltage is alternated at an ultrasonic frequency F, the piezoelectric material 303 will vibrate at the ultrasonic frequency F. The vibrations of the piezoelectric material 303 produce ultrasonic sound waves. Since the electrodes are cylindrically symmetric, the piezoelectric material 303 will vibrate radially, with cylindrical symmetry. Conversely, when an ultrasonic wave hits the piezoelectric material 303, the ultrasonic wave will cause vibrations in the piezoelectric material.

These vibrations will generate a voltage between the electrodes 305 and 304. Thus, the transducer is a reciprocal device that can both transmit and receive ultrasonic waves.

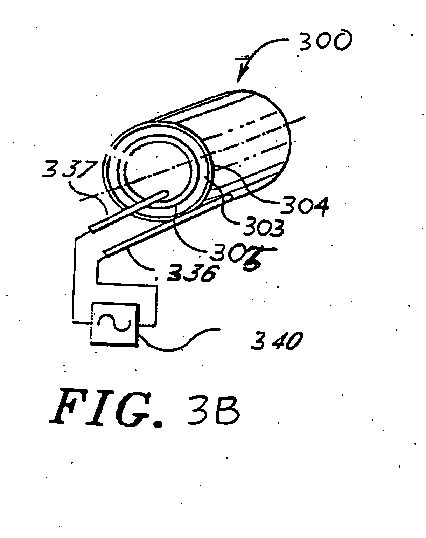

A detailed construction for a cylindrical ultrasound transducer is shown in Figure 3B. The length of the individual transducer 300 or ablation element 120 (e.g., multielement array of transducer elements 300) desirably is selected for a given clinical application. In connection with forming circumferential condition blocks in cardiac or pulmonary vein wall tissue, the ablation element 120 length can fall within the range of approximately $203{,}2 \cdot 10^{-3}$ cm (80 mils) up to greater than $1003 \cdot 10^{-3}$ cm (395 mils), and preferably equals about $508 \cdot 10^{3}$cm to $749{.}3 \cdot 10^{-3}$ cm (200 mils to 295 mils). A ablation element 120 accordingly sized is believed to form a lesion of a width sufficient to ensure the integrity of the formed conductive block without undue tissue ablation. For other applications, however, the length can be significantly longer.

Likewise, the transducer 300 or ablation element 120 outer diameter desirably is selected to account for delivery through a particular access path (e.g., percutaneously and transeptally), for proper placement and location within a particular body space, and for achieving a desired ablation effect. In the given application within or proximate of the pulmonary vein ostium, each transducer 300 (i.e ablation element 120) preferably has an outer diameter within the range of about $177{.}8 \cdot 10^{-3}$ cm (70 mils) to greater than $254 \cdot 10^{-3}$ cm (100 mils). It has been observed that a transducer 300 with an outer diameter of about $203{,}2 \cdot 10^{-3}$ cm (80 mils) generates acoustic power levels approaching 20 Watts per centimeter radiator or greater within myocardial or vascular tissue, which is believed to be sufficient for ablation of tissue engaged by the outer balloon for up to about 3.5 cm (1.4 inches) outer diameter of the balloon. For applications in other body spaces, the transducer 300 may have an outer diameter within the range of about $101{.}6 \cdot 10^{-3}$ cm (40 mils) to greater than $304{.}8 \cdot 10^{-3}$ cm to $406{.}4 \cdot 10^{-3}$ cm (120 to 160 mils) (e.g., as large as $1016 \cdot 10^{-3}$ to $2032 \cdot 10^{-3}$ cm (400 to 800 mils) for applications in some body spaces).

The central crystal layer 303 of the transducer 300 has a thickness selected to produce a desired operating frequency. The operating frequency will vary of course depending upon clinical needs, such as the tolerable outer diameter of the ablation and the depth of heating, as well as upon the size of the transducer as limited by the delivery path and the size of the target site. As described in greater detail below, the transducer 300 in the illustrated application preferably operates within the range of about 5 MHz to about 20 MHz, and more preferably within the range of about 7 MHz to about 10 MHz. Thus, for example, the transducer can have a thickness of approximately $30{.}48 \cdot 10^{-3}$ cm (12 mils) for an operating frequency of about 7 MHz (i.e., a thickness generally equal to 1/2 the wavelength associated with the desired operating frequency).

The transducer 300 is vibrated across the wall thickness to radiate a circumferential, collimated acoustic energy beam in the radial direction. In three dimensions, this circumferential acoustic energy beam has a ring or doughnut shape and appearance. For this purpose the distal ends of electrical leads 336, 337 are electrically coupled to outer and inner tubular members or electrodes 304, 305, respectively, of the transducer 300, such as, for example, by soldering the leads to the metallic coatings or by resistance welding. In the illustrated device, the electrical leads are $10{.}16{-}20{.}38 \cdot 10^{-3}$ cm (4-8 mil or 0.004 to 0.008 inch diameter) silver wire or the like. The proximal ends of these leads are adapted to couple to an ultrasonic driver or actuator 340, which is schematically illustrated in Figure 3B.

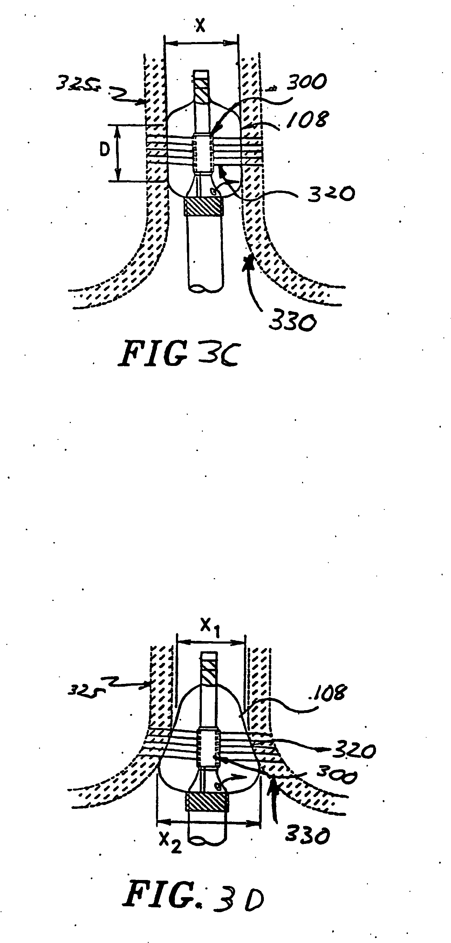

As previously described, in this configuration the acoustic energy remains highly collimated in the radial direction, and does not allow the acoustical beam to be projected forward or backward. This configuration also does not enable the operator to create an amplitude profile to focus the energy in an area of treatment i.e., at a particular distance from the transducer surface. Figures 3C and 3D illustrate two dimensional views of the radial acoustical energy beam paths 320 when the ablation device is placed in a pulmonary vein 325 and pulmonary vein ostium 330, respectively.

The present invention utilizes a tissue ablation element and device assembly capable of creating a circular energy beam that can be phased in the longitudinal direction, orienting the beam forward or backward. The present invention can also produce an acoustical energy beam having an additive effect at a predetermined distance from the ablation element 120.

In one embodiment of the invention the ablation element comprises a plurality of thin wall ultrasonic transducers serially arranged and coaxially disposed along a longitudinal axis to form a phased array. By controlling the driving power, operating frequency, and relative phase to each individual transducer in the array, the ultrasonic driver can focus acoustic energy at a fixed distance from the ablation element. In addition, the ultrasonic deriver can project the acoustic energy beam forward or backward to ablated the desired region of tissue.

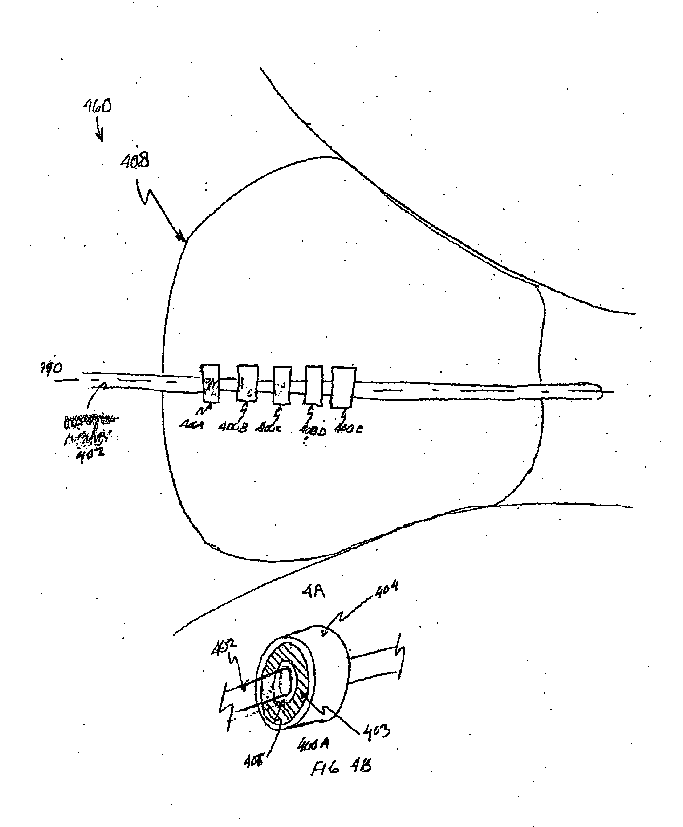

Figure 4A is a side view showing the construction of an ablation catheter 460 having a serially arranged array of ultrasonic transducers. The array is made from five (5) tube shaped piezoelectric transducers 400 (400a through 400e) mounted on a delivery member 402 along a longitudinal axis 490. Each transducer 400 (400a through 400e) comprises a piezoelectric crystal 403 between an inner electrode 405, and an outer electrode 404. Each transducer 400a through 400e has an outside diameter of approximately $254 \cdot 10^{-3}$ cm (100 mils), and a wall thickness of approximately $45{,}72 \cdot 10^{-3}$ cm (18 mils). The transducers 400a through 400e are spaced 2 millimeters apart. Figure 4B shows a perspective view of a typical transducer (400a) mounted on the delivery member 402.

The ablation catheter 460 also has an expandable member 408 to assist in locating and/or anchoring the ablation catheter 460 at the body location for ablation. In the illustrated embodiment, the expandable member 408 is a balloon similar to those earlier described, and the body location for ablation is a pulmonary vein ostium in a heart.

The number and spacing of the transducers 400 (400a through 400e) illustrated is exemplary. One of skill in the art would understand that other configurations are contemplated having more or fewer transducers 400. Similarly, the transducers 400 can be spaced along the delivery member 402 at different intervals. Several factors, including the desired application, may contribute to these and other configurations.

Each individual transducer 400 outer electrode 404 has an element pad (not shown) that serves as a connection point for the lead wires (not shown) used to energize the individual transducer 400a through 400e respectively. Each of these element pads.is substantially electrically insulated from one another to limit interference between individual transducers 400a through 400e. In addition, a ground pad (not shown) is attached to the inner electrode 405 of each transducers 400a through 400e and provides a connection point for a ground wire. Alternatively, the inner electrode 405 may be common to all the transducers 400a through 400e and be part of the delivery member 402.

Preferably, attachment of the lead and ground wires is by soldering the wires directly to the element and ground pads respectively. When an electrical potential is impressed across a particular end pad associated with a given transducer 400 and the ground pad, the transducer (400a through 400e) associated with the particular end pad is energized.

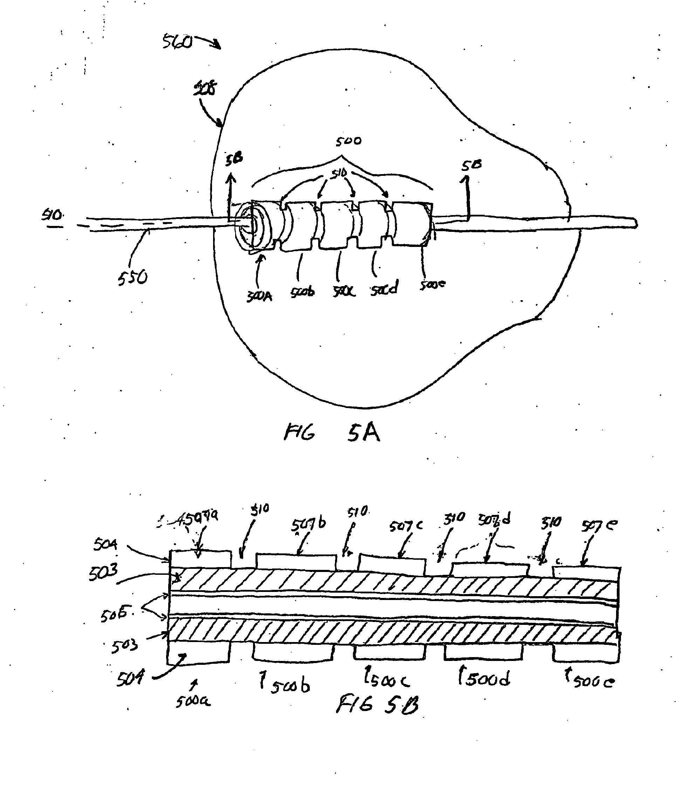

The serial array ablation element may also be created by segmenting a single long ultrasonic transducer into an array of substantially electrically insulated, functionally discrete transducer elements. Figure 5A is a perspective view of an ablation catheter having a single transducer 500 segmented into five (5) electrically insulated transducer elements 500a through 500e by grooves or notches 510 according to one embodiment of the present invention. Figure 5B is a section view, taken along line 5B - 5B in Figure 5A showing the cross-sectional area of the transducer 500 and transducer elements 500a through 500e.

The transducer 500 has an inner electrode 505 as a common electrode, and a cylindrical piezoelectric material 503 as a common element. The outer electrode 504 is segmented by grooves 510 into 5 individual electrodes 507 (507a through 507e) coaxially arranged along the outer transducer 500 longitudinal axis. The electrodes 507a through 507e are substantially electrically isolated from one another and correspond to the array of five transducers elements 500a through 500e.

When AC voltage is impressed between the inner electrode 505 and a selected one of the five outer electrode 504 segments (507a - 507e), the piezoelectric material vibrates in the region between the inner electrode 505 and the selected outer electrode segment 507. For example, an AC voltage impressed between the inner electrode 505 and outer electrode element 507b will cause the region between the electrode 505 and the electrode segment 507b to vibrate. However, the piezoelectric material 503 is a single piece of un-sectioned material as shown in Figures 5A and 5B, so the impressed voltage and subsequent vibration between the inner electrode 505 and the outer electrode element 507b will cause some vibration in the regions between the inner electrode 505 and outer electrode segments 507a and 507c adjacent to electrode segment 507b. This coupling of signals is sometimes referred to a cross-talk.

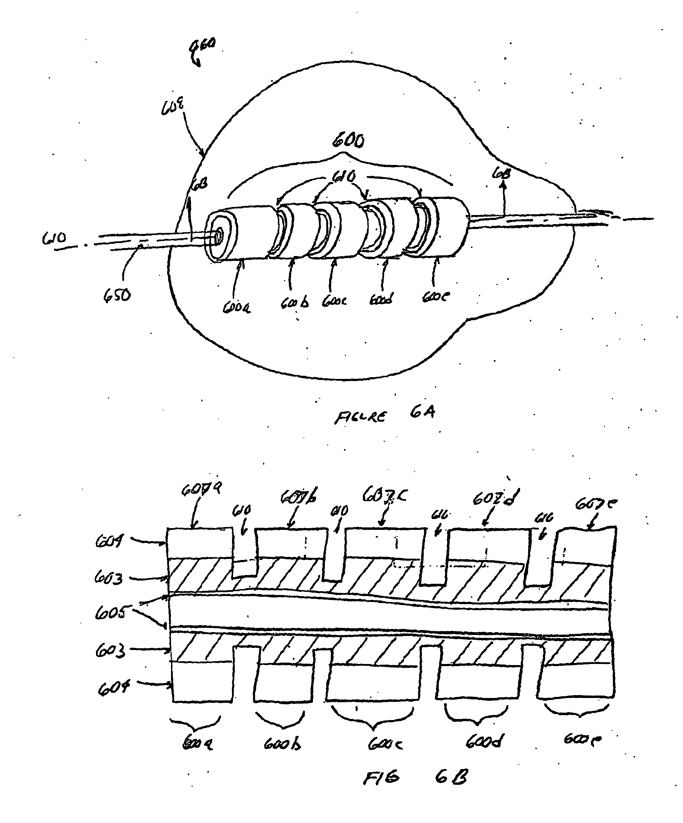

Excessive cross-talk between electrodes may be undesirable for some particular applications. To reduce such coupling between adjacent electrodes, the elements may be partially isolated from one another. Figures 6A and 6B are perspective and section views respectively showing the construction of an ablation catheter 660 having an ablation element comprising transducer 600 with grooves extending into the cylindrical piezoelectric material 603 mounted on an ablation catheter 650 according to one embodiment of the present invention. By extending the grooves into the piezoelectric material 603, the piezoelectric material 603 will be zoned, partially isolating the signals and subsequently reducing cross-talk. Figure 6A also shows an expandable member 608 for locating and/or anchoring the ablation element at the target area.

As similarly described above, transducer 600 is constructed having grooves 610 sectioning transducer 600 into an array of serially arranged discrete transducer elements 600a through 600e. The transducer 600 has an inner electrode 605 as a common electrode, and a cylindrical piezoelectric material 603 at least partially as a common element. The outer electrode 604 is separated by circumferential grooves 610 into 5 individual electrode segments 607 (607a through 607e) circumferentially disposed around the outer transducer 600 surface. These electrode segments 607a through 607e directly correspond to transducer elements 600a through 600e. However, unlike the transducer 500 illustrated in Figures 5A and 5B, grooves 610 radially extend completely through the outer electrode and into at least a portion of the cylindrical piezoelectric material 603. The grooves in the piezoelectric material 603 will tend to physically separate the piezoelectric material 603 into zones (five zones in the illustrated embodiment) directly corresponding to the five electrode segments 607a through 607e.

The coupling between the electrodes can be further reduced by extending the grooves 610 all the way through the piezoelectric material (not shown), thereby producing separate pieces of piezoelectric material, and thus completely separate transducers.

Segmentation of the transducer 500, 600 may be accomplished by etching or notching grooves into at least the outer electrode 504, 604 of transducer 500, 600, separating the transducer 500, 600 into functionally discrete transducer elements (500a through 500e), (600a through 600e). The grooves can be made using several different methods known in the art, such as for example etching using a diamond wheel or laser. One particular laser machining method that may be adapted to cut grooves is disclosed by Corbett, Scott et al. in "Laser Machining of High Density Two-Dimensional Ultrasound Arrays" (2002), which is incorporated by reference in its entirety herein. This method uses a YAG laser emitting a wavelength of 355nm to essentially etch or evaporate the material and create the elements 507, 607. Other machining methods capable of achieving the desired configuration, such as those used to laser etch stents and other medical devices, may be used and are known in the art.

In a preferred embodiment a Nd-YAG laser is coupled with a CNC system accurate to within a few microns to cut the pattern. The grooves etched or notched by the laser are approximately $7.62 \cdot 10^{-3}$ cm (3 mils) deep and $5.08 \cdot 10^{-3}$ cm (2 mils) wide. The element end pads and ground pad may be similarly formed using the laser and CNC machine.

The plurality of transducers (400a through 400e, 500a through 500e, 600a through 600e), may be operated in at least three modes. In a first mode, all five transducers or transducer segments (simulating five serially arranged transducers) are driven with identical signals. This mode will create a single radial acoustic energy beam having a radial thickness similar to existing single transducer designs. In a second mode, the five individual transducers or transducer segments are driven as a standard phased array by signals having a fixed phased delay between segments. The phased array allows the resultant energy beam to be directed forward or backward. In still a third mode, each transducer is driven by a signal that has a common frequency, but each transducer or segment has a phase which is chosen to give an additive effect at a predetermined distance from the transducer. This generates an amplitude profile to focus the cumulative energy beam in the area of treatment while minimizing the effect on nearby anatomical features.

A phased delay is a representation of the *time delay* in seconds experienced by each sinusoidal component of the input signal. The phase of a periodic phenomenon i.e. sinusoidal input signal, can also be expressed or specified by angular measure, with one period usually encompassing 360° ($2\pi$ radians). When each transducer element is driven at the same frequency, the phase delay will be directly related to the phase shift or the change In phase angle between each sinusoidal component of the input signal.

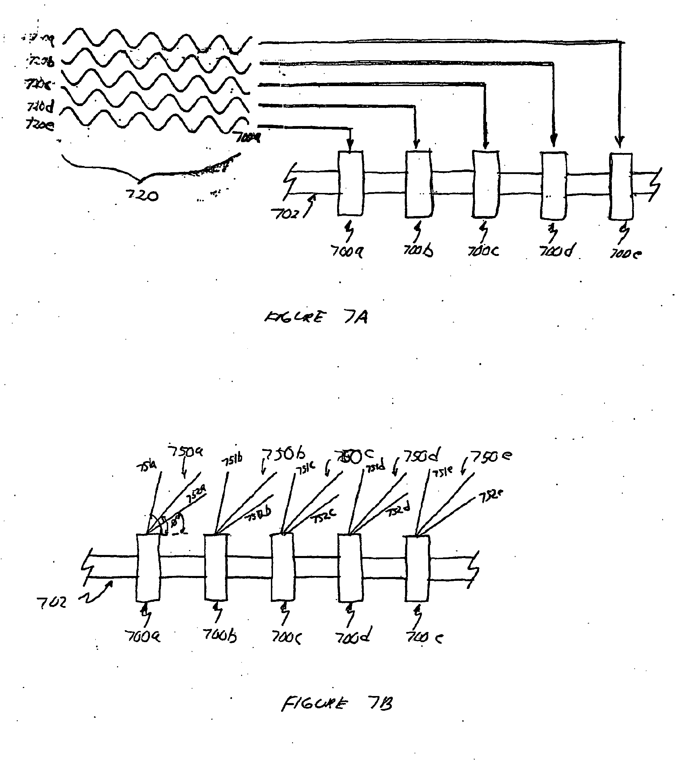

A schematic representation illustrating a fixed phase delay (phase shift) for a plurality of sinusoidal input signals 720 (720a through 720e) driving an array of transducers 700a through 700e is shown in Figure 7A. This design utilizes five transducers driven through a five-channel generator with five leads. In the illustrated schematic, like reference numerals are used to show the association between particular fixed phase input signals 720a through 720e, transducers 700a through 700e, and output energy signals 750a through 750e. For example, transducer 700a produces sinusoidal ultrasonic sound wave 750a.

When an alternating sinusoidal input current 720a through 720e is impressed between the outer electrode 704 and inner electrode 705 of a particular transducer 700, the thickness of the piezoelectric material 703 associated with the given transducer 700 (700a through 700e) will vibrate at the alternating frequency. The repetitive cyclic design illustrated in Figure 7A produces an array that has the same signal every fifth element. Accordingly, the total cumulative phase shift over the five transducers 700a through 700e, is equal to a full 360 degrees. Using a fixed phase delay, the optimal phase shift between adjacent transducer segments (700a through 700e) is thus 72 degrees. As can be seen from the illustrated embodiment, input signal 720a is 72 degrees out of phase from input signal 720b. Similarly, input signal 720b is 72 degree out of phase from input signal 720c, and so on. This configuration maximizes transducer efficiency and provides a coherent energy beam.

Typically, a cylindrical ultrasound transducer will produce a highly collimated acoustic energy beam that emanates from the transducer in a direction substantially normal to the transducer longitudinal axis. When the transducers are circular in cross-section, as depicted in Figures 3 through 6, this acoustic energy beam radiates out from the transducer surface is ring shaped, covering a full 360 degrees. Similarly, a plurality of transducers arranged serially along a longitudinal axis would produce a highly collimated acoustic energy beam normal to the transducers longitudinal axis when the individual transducers are driven in-phase with respect to one another. However, when the transducers are driven out of phase from one another, as illustrated in Figure 7B, the resultant cumulative acoustic energy beam emanates from the transducer 700 (700a - 700e) at an angle relative to the longitudinal axis. By varying the phase delay of the input signal 720, the acoustical energy beam angle will change. The implication is that for a different acoustic energy beam angle, a different phase delay would be used.

By varying the phase of the input signal to the transducers, that is, providing transducers with varying relative input signal phases, the acoustic energy beam ring can be focused on a particular anatomical location, and at a particular distance from the transducers. For example, the relative phases between transducer input signals may be programmed to focus the acoustic energy beam at a location that corresponds to the diameter of the expandable member, such as the expandable balloon, to ensure that the treated area is a fixed distance from the outside of the balloon.

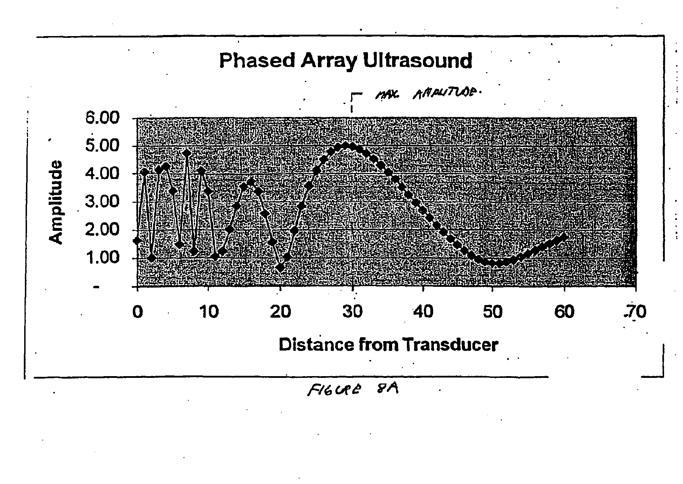

Figure 8A is a graphical representation of an amplitude profile for an acoustical energy beam emanating from an array of transducers according to one embodiment of the present invention. As previously disclosed, each transducer is driven by an input signal that has a common frequency. However, each transducer input signal has a phase that is chosen to produce a cumulative acoustic energy beam having an additive or amplified effect at a predetermined distance from the transducer. This generates an amplitude profile that will focus the energy in the area of treatment while minimizing the effect on nearby anatomical features.

The phase shift or time delay between the input signals for each transducer In the phased array are directly related to the location of the focal point for the acoustic energy beam. This time delay is equal to the time is takes the ultrasonic energy beam to travel the distance from each particular transducer in the array to the target site. The relationship between the time delay and distance can be defined using the following formula:

$$t = L/V$$

where:

- t is the time it takes the acoustic energy beam to travel from the particular transducer to the target site;
- L is the distance from the particular transducer to the target site; and
- V is the speed of sound in water, approximately 1550 meters/second.

This time (t) it takes the acoustic energy beam to travel from the transducer to the target site is calculated for each transducer in the array. The desired time delay ($t_d$) is just the difference between acoustic energy beam travel times of the particular transducers being activated in the array.

To express the time delay as function of phase you simply multiply the time delay by the frequency using the following formula:

$$PH = 2\pi f t$$

where:

- PH is the phase in radians;
- $f$ is the frequency of the input signal, in Hertz; and
- $t_d$ is the time delay in seconds.

One of skill in the art would understand that there are $2\pi$ radians per angular degree. Accordingly, to represent the phase in degrees, the ($2\pi$) term in the formula above should be substituted with 360 degrees.

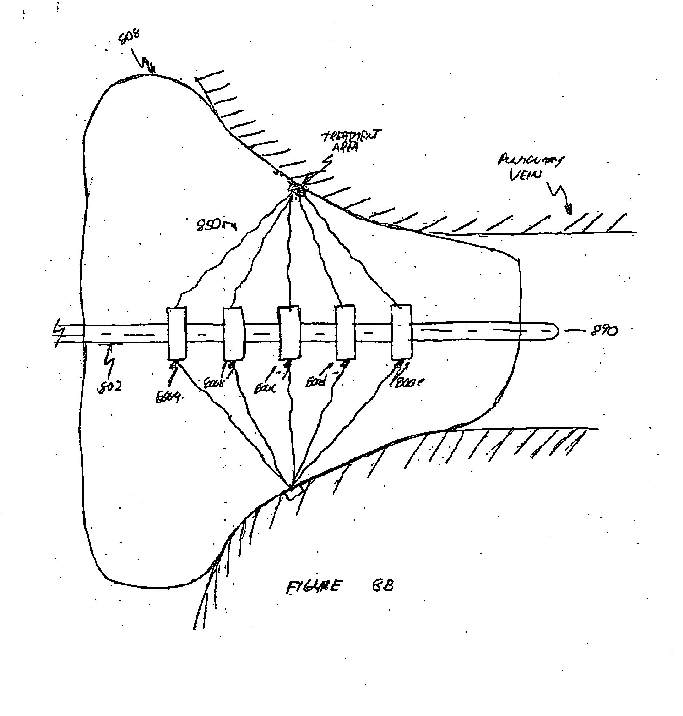

To generate the amplitude profile illustrated in Figure 8A, five (5) tube shaped piezoelectric transducers 800 (800a through 800e) were mounted two (2) millimeters apart on a delivery member 802 along a longitudinal axis 890 as illustrated in Figure 8B. Each transducer 800 (800a through 800e) comprises a piezoelectric crystal 803 between an inner electrode 805, and an outer electrode 804. The transducers 800a through 800e were each driven at a frequency of 8Mhz, with relative phases of -60 degrees, -30 degrees, 90 degrees, -30 degrees, and -60 degrees respectively. As can be seen from the profile illustrated in Figure 8A, this configuration provides a maximum amplitude at a distance of 30mm from the transducer, which corresponds to the diameter of the expandable balloon 808 at the desired treatment site illustrated in Figure 8B. This ensures that the acoustical energy beam 850 is focused on the treatment area at a fixed distance outside of the balloon.

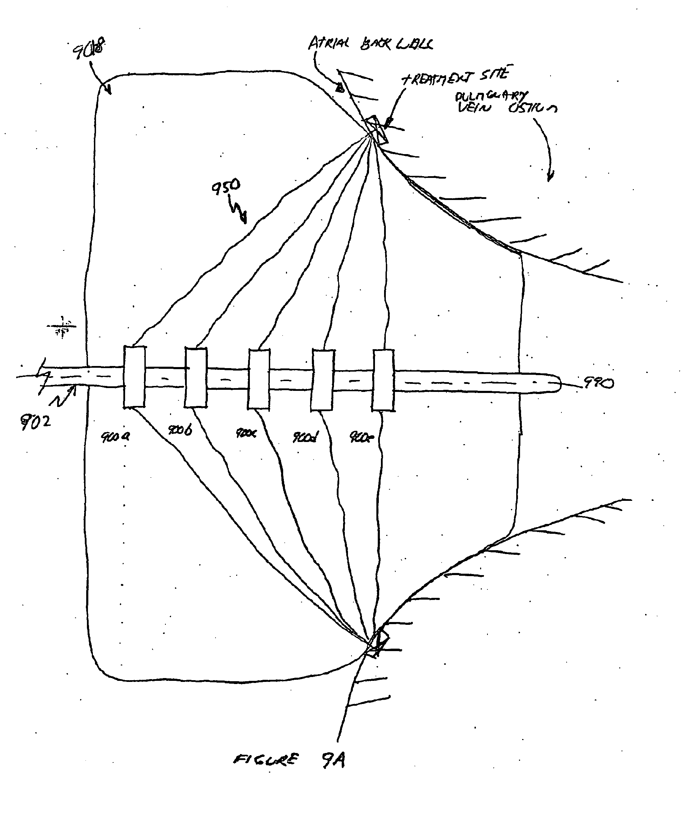

Similarly, the transducers may be phased to focus the energy beam forward or backward, to a ring that lies in a plane, which is not in the center of the middle transducer. Figure 9A is a schematic representation showing the construction of an ablation catheter 960 having an ablation element comprising an array of five (5) serially arranged ultrasonic transducers 900a - 900e. The transducers 900a - 900e are mounted on a delivery member 902 along a longitudinal axis 990. The ablation catheter 960 also has an expandable member 908 to assist in locating and anchoring the ablation catheter at the body location for ablation. By operating the ultrasonic transducers 900a - 900e at a common frequency, but varying the phase (phase shift) between adjacent transducers, the acoustic energy beam can be focused in a ring in a forward (as shown) or backward direction. Varying the phase shift will allow the angle of the resultant acoustic energy beam to greater or smaller, effectively locating the acoustic energy beam at the target location.

Another method to vary the phase delay is to vary the frequency at which the transducers are driven while keeping the phase shift (angle) between adjacent input signals the same.

As noted above, an acoustical energy beam can be projected at an angle 90° (i.e. perpendicular) to the longitudinal axis with any frequency in the transducer's bandwidth by driving all the segments comprising the transducer in-phase with one another. In addition, the illustrated array of transducer segments can also be driven with phase delays that are not fixed, or would not sum to 360° as previously disclosed.

Several factors should be considered when selecting a generator to produce the acoustic energy beam. The generator should have at least one channel for each electrode element (i.e. for each transducer segment). Using the illustrated embodiment as an example, the generator would be, as a minimum, a five-channel signal generator with an amplifier output stage capable of phase-lock operation. A linear RF amplifier should be provided for each channel matched for driving a 50 Ohn load up to 20 Watts per channel. The amplifiers should have a bandwidth of up to 12 MHz and should have identical gain and phase shift across the channels. The generator should preferably have directional couplers, shunt resistors to dissipate reflected power, and sensing circuits for reflected power magnitude and phase.

Preferably, the signal generator would be a computer driven signal generator capable of generating highly coherent continuous sine wave signals with accurate phase delay between the channels. The computer should be capable of obtaining the desired angle as an input, and calculate the frequency and phase for each of the five channels. Other desirable inputs to the computer should include the desirable output power, the direct and reflected power of each channel, and the target tissue temperature. If the transducer is also going to be used for imaging, appropriate considerations should be taken into the design of the generator, such as the ability to generate short bursts of acoustic energy with accurate timing.

The foregoing invention variously shows circumferential ablation device assemblies incorporating ultrasound transducers for ablating a circumferential region of tissue. Such ultrasound ablation assemblies are believed to be particularly amenable to use with position monitoring assemblies incorporating sensing capabilities of the ablation transducer itself, such as for example but not limited to an "A"-mode sensing system. However, it is further contemplated that the particular ablation devices may also be combined with the other position monitoring assemblies and related sensors. Furthermore, such ultrasound ablation assemblies may also be combined with the various ablation monitoring assemblies, such as temperature monitoring assemblies and sensors.

As common to each of the following devices, a source of acoustic energy is provided with a delivery device that may also includes an anchoring mechanism. In one mode, the anchoring device comprises an expandable member that also positions the acoustic energy source within the body; however, other anchoring and positioning devices may also be used, such as, for example, a basket mechanism.

In a more specific form, the acoustic energy source is located within the expandable member and the expandable member is adapted to engage a circumferential path of tissue either about or along a pulmonary vein in the region of its ostium along a left atrial wall. Prior art acoustic energy sources in turn are acoustically coupled to the wall of the expandable member and thus to the circumferential region of tissue engaged by the expandable member wall by emitting a circumferential and longitudinally collimated ultrasound signal when actuated by an acoustic energy driver. The use of acoustic energy, and particularly ultrasonic energy, offers the advantage of simultaneously applying a dose of energy sufficient to ablate a relatively large surface area within or near the heart to a desired heating depth without exposing the heart to a large amount of current For example, an ultrasonic transducer can form a lesion, which has about a 1.5 mm width, about a 2.5 mm diameter lumen, such as a pulmonary vein and of a sufficient depth to form an effective conductive block. It is believed that an effective conductive block can be formed by producing a lesion within the tissue that is transmural or substantially transmural. Depending upon the patient as well as the location within the pulmonary vein ostium, the lesion may have a depth of 1 millimeter to 10 millimeters. It has been observed that the ultrasonic transducer can be powered to provide a lesion having these parameters so as to form an effective conductive block between the pulmonary vein and the posterior wall of the left atrium.

While particular detailed description has been herein provided for particular embodiments and variations according to the present invention, it is further understood that various modifications and improvements may be made by one of ordinary skill according to this disclosure and without departing from the broad scope of the invention.

In addition, a circumferential ablation device assembly constructed with a mounted ultrasound ablation element according to the present invention may be used in combination with other linear ablation assemblies and methods, and various related components or steps of such assemblies or methods, respectively, in order to form a circumferential conduction block adjunctively to the formation of long linear lesions, such as in a less-invasive "maze"-type procedure.

In addition, one of ordinary skill may make other obvious or insubstantial modifications or improvements to the specific embodiments herein shown and described based upon this disclosure without departing from the scope of the invention as defined by the claims that follow.

## Claims

1. A cylindrical ultrasound transducer (500; 600) comprising:

   a cylindrical inner electrode (505; 605);
   a cylindrical piezoelectric material (503; 603) disposed over the inner electrode (505; 605); and
   a cylindrical outer electrode (504; 604) disposed over the cylindrical piezoelectric material, the cylindrical outer electrode (504; 604) having grooves (510; 610) **characterized in that** the grooves (510; 610) are circumferential, wherein each groove (510; 610) starts at one location, translates along the circumference of the outer electrode (504; 604) and is completed at the starting location, and the grooves (510; 610) separate the outer electrode (504; 604) into a plurality of discrete serially arranged transducer elements (500a-500e; 600a-600e).

2. The cylindrical ultrasound transducer (500; 600) of claim 1 wherein the inner electrode (505; 605) comprises a metallic layer.

3. The cylindrical ultrasound transducer (500; 600) of claim 1 wherein the cylindrical piezoelectric material (503; 603) comprises a high-density fine grain PZT ceramic material.

4. The cylindrical ultrasound transducer (500; 600) of claim 1 wherein the cylindrical piezoelectric material (503; 603) is polished to a mirror finish of approximately 10 microns.

5. The cylindrical ultrasound transducer (500; 600) of claim 1 wherein the outer electrode (504; 604) comprises a metallic layer.

6. The cylindrical ultrasound transducer (500; 600) of claim 2 or 5 wherein the metallic layer comprises Nickel.

7. The cylindrical ultrasound transducer (500; 600) of claim 2 or 5 wherein the metallic layer comprises Gold.

8. The cylindrical ultrasound transducer (600) of claim 1, wherein the circumferential grooves (610) cut through the outer electrode (604) and at least a portion of the cylindrical piezoelectric material (603).

## Patentansprüche

1. Zylindrischer Ultraschall-Transducer (500; 600), der aufweist:

   eine zylindrische innere Elektrode (505; 605);
   ein zylindrisches piezoelektrisches Material (503; 603), das über der inneren Elektrode (505; 605) angeordnet ist; und
   eine zylindrische äußere Elektrode (504; 604), die über dem zylindrischen piezoelektrischen Material angeordnet ist, wobei die zylindrische äußere Elektrode (504; 604) Nuten (510; 610) hat,
   **dadurch gekennzeichnet, dass** die Nuten (510; 610) am Umfang verlaufen, wobei jede Nut (510; 610) an einem Ort beginnt, sich entlang dem Umfang der äußeren Elektrode (504; 604) erstreckt und am Ort des Beginns endet, und die Nuten (510; 610) die äußere Elektrode (504; 604) in eine Vielzahl getrennter seriell angeordneter Transducerelemente (500a-500e; 600a-600e) trennt.

2. Zylindrischer Ultraschall-Transducer (500; 600) nach Anspruch 1, bei dem die innere Elektrode (505; 605) eine metallische Schicht aufweist.

3. Zylindrischer Ultraschall-Transducer (500; 600) nach Anspruch, bei dem das zylindrische piezoelektrische Material (503; 603) ein hochdichtes, feinkörniges PZT-Keramikmaterial aufweist.

4. Zylindrischer Ultraschall-Transducer (500; 600) nach Anspruch 1, bei dem das zylindrische piezoelektrische Material (503; 603) auf einen Spiegelglanz von ungefähr 10 Mikrometern poliert ist.

5. Zylindrischer Ultraschall-Transducer (500; 600) nach Anspruch 1, bei dem die äußere Elektrode (504; 604) eine metallische Schicht aufweist.

6. Zylindrischer Ultraschall-Transducer (500; 600) nach Anspruch 2 oder 5, bei dem die metallische Schicht Nickel aufweist.

7. Zylindrischer Ultraschall-Transducer (500; 600) nach Anspruch 2 oder 5, bei dem die metallische Schicht Gold aufweist.

8. Zylindrischer Ultraschall-Transducer (600) nach Anspruch 1, bei dem die am Umfang verlaufenden Nuten (610) die äußere Elektrode (604) und wenigstens einen Teil des zylindrischen piezoelektrischen Materials (603) durchschneiden.

**Revendications**

1.  Transducteur ultrasonore cylindrique (500 ; 600) comprenant :

    ✔ une électrode interne cylindrique (505 ; 605) ;
    ✔ un matériau piézoélectrique cylindrique (503 ; 603) disposé sur l'électrode interne (505 ; 605) ; et
    ✔ une électrode externe cylindrique (504 ; 604) disposée sur le matériau piézoélectrique cylindrique, l'électrode externe cylindrique (504 ; 604) ayant des rainures (510 ; 610), **caractérisé en ce que** les rainures (510 ; 610) sont circonférentielles, chaque rainure (510 ; 610) commençant à un certain emplacement, s'étendant sur la circonférence de l'électrode externe (504 ; 604) et rejoignant l'emplacement initial, et les rainures (510 ; 610) séparant l'électrode externe (504 ; 604) en une pluralité d'éléments transducteurs (500a-500e ; 600a-600e) agencés en série de manière discrète.

2.  Transducteur ultrasonore cylindrique (500 ; 600) selon la revendication 1, dans lequel l'électrode interne (505 ; 605) comprend une couche métallique.

3.  Transducteur ultrasonore cylindrique (500 ; 600) selon la revendication 1, dans lequel le matériau piézoélectrique cylindrique (503 ; 603) comprend un matériau céramique PZT à grains fins haute densité.

4.  Transducteur ultrasonore cylindrique (500 ; 600) selon la revendication 1, dans lequel le matériau piézoélectrique cylindrique (503 ; 603) est poli jusqu'à obtenir un fini miroir d'approximativement 10 microns.

5.  Transducteur ultrasonore cylindrique (500 ; 600) selon la revendication 1, dans lequel l'électrode externe (504 ; 604) comprend une couche métallique.

6.  Transducteur ultrasonore cylindrique (500 ; 600) selon la revendication 2 ou 5, dans lequel la couche métallique comprend du nickel.

7.  Transducteur ultrasonore cylindrique (500 ; 600) selon la revendication 2 ou 5, dans lequel la couche métallique comprend de l'or.

8.  Transducteur ultrasonore cylindrique (600) selon la revendication 1, dans lequel les rainures circonférentielles (610) coupent l'électrode externe (604) et au moins une partie du matériau piézoélectrique cylindrique (603).

**FIG. 1A**

**FIG. 1B**

**FIG. 1C**

**FIG. 1D**

EP 1 791 482 B1

POSITION MONITORING SYSTEM

DISPLAY

ABLATION CONTROL SYSTEM

FIG.2A

*FIG.2B*

FIG. 3A

FIG. 3B

X

325 → 300
108
D
320

330

FIG 3C

X₁

325 → 108
320
300

330

X₂

FIG. 3D

460

408

190

402

400A 400B 400C 400D 400E

4A

404

402

403

406

400A

FIG 4B

EP 1 791 482 B1

FIG 5A

FIG 5B

23

FIGURE   6A

FIG    6B

24

720a
720b
720c
720d
720e

720

702

700a  700b  700c  700d  700e

FIGURE 7A

750a  750b  750c  750d  750e
751a  751b  751c  751d  751e
752a  752b  752c  752d  752e

702

700a  700b  700c  700d  700e

FIGURE 7B

FIGURE 8A

FIGURE 8B

FIGURE 9A

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4672962 A, Hershenson
- US 4676258 A, InoKuchi
- US 4790311 A, Ruiz
- US 4807620 A, Strul
- US 4998933 A, Eggers
- US 5035694 A, Kasprzyk
- US 5190540 A, Lee
- US 5226430 A, Spears
- US 5292321 A, Lee
- US 5449380 A, Chin
- US 5505730 A, Edwards
- US 5558672 A, Edwards
- US 5562720 A, Stem
- US 4449528 A, Auth
- US 4522205 A, Taylor
- US 4662368 A, Hussein
- US 5078736 A, Behl
- US 5178618 A, Kandarpa
- US 5348554 A, Imran
- US 5423811 A, Imran
- US 5545161 A, Imran
- US 5569241 A, Edwards
- US 5575788 A, Baker
- US 5658278 A, Imran
- US 5688267 A, Panescu
- US 5697927 A, Imran
- US 5722403 A, McGee
- US 5769846 A
- WO 9732525 A, Pomeranz
- WO 9802201 A, Pomeranz
- US 4641649 A, Walinsky
- WO 9632897 A, Desai

- US 4673563 A, Berne
- US 4569801 A, Molloy
- US 4898591 A, Jang
- US 5104393 A, Isner
- US 5427119 A
- US 5487385 A
- US 5497119 A, Swartz
- US 5545193 A, Fleischman
- US 5549661 A, Kordis
- US 5575810 A, Swanson
- US 5564440 A, Swartz
- US 5592609 A, Swanson
- US 5575766 A, Swartz
- US 5582609 A, Swanson
- US 5617854 A, Munsif
- US 5687723 A, Avitall
- US 5702438 A, Avitall
- WO 9320767 A, Stem
- WO 9421165 A, Kordis
- WO 9610961 A, Fleischman
- WO 9626675 A, Klein
- WO 9737607 A, Schaer
- US 5971983 A
- WO 9902096 A, Diederich
- US 6024740 A, Michael D. Lesh
- US 6012457 A, Michael D. Lesh
- US 6117101 A, Chris J. Diederich
- US 09260316 B
- US 5938660 A
- WO 9900064 A
- US 4135109 A
- WO 2005009218 A

### Non-patent literature cited in the description

- **Hindricks.** *Current Management of Arrhythmias,* 1991
- **Cox, J. L et al.** The surgical treatment of atrial fibrillation. I. Summary. *Thoracic and Cardiovascular Surgery,* 1991, vol. 101 (3), 402-405
- **Cox, J L.** The surgical treatment of atrial fibrillation. IV. Surgical Technique. *Thoracic and Cardiovascular Surgery,* 1991, vol. 101 (4), 584-592
- **Sueda et al.** *Simple Left Atrial Procedure for Chronic Atrial Fibrillation Associated With Mitral Valve Disease,* 1996

- **Avitall et al.** Physics and Engineering of Transcatheter Tissue Ablation. *Journal of American College of Cardiology,* 1993, vol. 22 (3), 921-932
- **Haissaguerre et al.** Right and Left Atrial Radiofrequency Catheter Therapy of Paroxysmal Atrial Fibrillation. *Journal of Cardiovascular Electrophysiology,* 1996, vol. 7 (12), 1132-1144
- **Fram et al.** Feasibility of RF Powered Thermal Balloon Ablation of Atrioventricular Bypass Tracts via the Coronary Sinus: In vivo Canine Studies. *PACE,* 1995, vol. 18, 1518-1530

- **Schuger CD et al.** Long-term effects of percutaneous laser balloon ablation from the canine coronary sinus. *Circulation,* 1992, vol. 86, 947-954
- **McMath L P et al.** Percutaneous laser balloon coagulation of accessory pathways. *Diagn Ther Cardiovasc Interven,* 1991, vol. 1425, 165-171
- **Jais et al.** A focal source of atrial fibrillation treated by discrete radiofrequency ablation. *Circulation,* 1997, vol. 95, 572-576
- **Corbett, Scott et al.** *Laser Machining of High Density Two-Dimensional Ultrasound Arrays,* 2002